# EUROPÄISCHE PATENTSCHRIFT

(11) **EP 2 604 231 B1**
(45) Veröffentlichungstag und Bekanntmachung des Hinweises auf die Patenterteilung: **06.04.2016**
(21) Anmeldenummer: 12192569.7
(22) Anmeldetag: 14.11.2012
(51) Int. Cl.: A61F 2/95, A61F 2/966

(54) **Freigabevorrichtung zum Lösen eines medizinischen Implantats von einem Katheter sowie Katheter mit einer Freigabevorrichtung**
Release mechanism for releasing a medical implant from a catheter, and catheter having a release mechanism
Dispositif de libération pour libérer un implant médical d'un cathéter ainsi que cathéter avec dispositif de libération

(30) Priorität: 12.12.2011 US 201161569299 P
(43) Veröffentlichungstag der Anmeldung: 19.06.2013
(73) Patentinhaber: Biotronik AG, 8180 Bülach (CH)
(72) Erfinder: Fargahi, Amir, 8180 Bülach (CH)
(74) Vertreter: Galander, Marcus

(56) Entgegenhaltungen:
- WO-A1-2011/094527
- US-A- 5 601 568
- US-A1- 2002 004 663
- US-A1- 2002 120 277
- US-A1- 2003 028 236
- US-A1- 2003 225 445
- US-A1- 2007 118 201

## Beschreibung

Die Erfindung betrifft eine Freigabevorrichtung zum Lösen eines medizinischen Implantats von einem Katheter sowie einen Katheter mit einer Freigabevorrichtung zum Freigeben eines medizinischen Implantats zur Implantation in einen tierischen und/oder menschlichen Körper nach den Oberbegriffen der unabhängigen Patentansprüche.

In der Medizin kommen häufig Implantate zum Einsatz, die zur Erfüllung von Ersatzfunktionen permanent oder zumindest für einen längeren Zeitraum in einen tierischen und/oder menschlichen Körper eingebracht werden. Zu nennen wären hier beispielsweise Herzschrittmacher, Hirnschrittmacher für Parkinsonpatienten, Herzimplantate, Cochleaimplantate, Retina Implantate, zahnmedizinische Implantate, Implantate zum Gelenkersatz, Gefäßprotesen oder Stents.

Implantate sind zum Einführen in den Körper mit Kathetern verbunden und müssen am Einsatzort genau platziert und definiert freigegeben werden können. Hierzu ist beispielsweise bekannt, das Implantat durch eine Schiebbewegung freizugeben.

US 2003/0225445 beschreibt eine Vorrichtung zum Laden eines selbstexpandierenden Stents in den Aufnahmebereich einer Einführvorrichtung.

Der Erfindung liegt die Aufgabe zugrunde, eine Freigabevorrichtung anzugeben, mit der ein hochpräzises und gezieltes Freigeben eines Implantats erfolgen kann.

Eine weitere Aufgabe ist in der Bereitstellung einer entsprechenden Einführvorrichtung zu sehen.

Die Aufgabe wird erfindungsgemäß durch die Merkmale der unabhängigen Ansprüche gelöst. Günstige Ausgestaltungen und Vorteile der Erfindung ergeben sich aus den weiteren Ansprüchen und der Beschreibung.

Es wird eine Freigabevorrichtung zum Lösen eines medizinischen Implantats von einer Einführvorrichtung vorgeschlagen, bei welcher das Implantat durch eine Relativbewegung zwischen einem ersten und einem zweiten Einführelement freisetzbar ist. Die Freigabevorrichtung umfasst einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende ein Aktor vorgesehen ist, wobei der Aktor zur Erzeugung einer gezielten Relativbewegung zwischen dem ersten und dem zweiten Einführelement der Einführvorrichtung eine erste und zumindest eine zweite Bewegungsrichtung aufweist, wobei bei der ersten Bewegungsrichtung das erste und das zweite Einführelemente in Longitudinalrichtung gegeneinander verschiebbar sind und wobei der Aktor bei der zumindest zweiten Bewegungsrichtung eine Bewegung quer zur Longitudinalrichtung bewirkt.

Durch die erfindungsgemäße Ausgestaltung kann eine Freigabevorrichtung bereitgestellt werden, bei der große Kräfte einfach erzeugt werden können. Dadurch kann eine vom Anwender und/oder Arzt zu erbringende Kraft bedienerfreundlich minimiert werden. Der Anwender und/oder Arzt kann sich auf eine korrekte Positionierung des Implantats konzentrieren. Die Freigabe des Implantats wird dadurch präziser und schneller. Ferner kann mittels des Aktors die Relativbewegung stabil und gleichmäßig realisiert werden. All dies resultiert in einer hohen Erfolgsrate der Implantation.

In diesem Zusammenhang soll unter einer Longitudinalrichtung eine Längsrichtung, eine Richtung in eine Längserstreckung und/oder eine longitudinale Verschieberichtung der Einführvorrichtung verstanden werden. Die erste Bewegungsrichtung ist bevorzugt eine Bewegung in Longitudinalrichtung. Unter dem Begriff "bewirken" soll "erzeugen, veranlassen und/oder erreichen" verstanden werden. Ferner soll unter "vorgesehen" speziell ausgestattet und/oder ausgelegt verstanden werden. Die Bewegungen in die erste und die zumindest zweite Bewegungsrichtung können zeitlich nacheinander und/oder bevorzugt zeitgleich erfolgen. Die Bewegung quer zur Longitudinalrichtung wird in ihrer Distanz durch die Breite der Freigabevorrichtung begrenzt (und hat z. B. einen Bewegungsraum von ca. 25 mm). Der Aktor kann vorteilhaft eine Bewegung quer zu der Longitudinalrichtung in eine Bewegung in Longitudinalrichtung umsetzen. Bevorzugt bewegt sich ein erstes Bauelement des Aktors in die erste und ein zweites, von dem ersten Bauelement unterschiedliches Bauelement des Aktors in die zumindest zweite Bewegungsrichtung.

Besonders vorteilhaft bewirkt der Aktor bei der zumindest zweiten Bewegungsrichtung eine Bewegung im Wesentlichen senkrecht und insbesondere senkrecht zur Longitudinalrichtung. Hierdurch kann der Aktor insbesondere behinderungsfrei zwei Bewegungen in zwei Bewegungsrichtungen vermitteln und/oder mit zwei unterschiedlichen Bewegungsrichtungen betrieben werden. Hierbei soll unter der Wendung "im Wesentlichen senkrecht" verstanden werden, dass auch eine Abweichung von bis zu 30° der zweiten Bewegungsrichtung gegenüber der Longitudinalrichtung als senkrecht verstanden werden soll.

Der Aktor weist zumindest eine Spindel auf, die in Richtung der zumindest zweiten Bewegungsrichtung angeordnet ist, wodurch diese konstruktiv einfach die zweite Bewegungsrichtung vorgeben kann, und/oder die die Bewegung quer zu der Longitudinalrichtung bewirkt. Durch diese Realisierung kann die Bewegung in die zumindest zweite Bewegungsrichtung mit einfachen Mitteln realisiert werden. Bevorzugt sind Bauelemente des Aktors während der Bewegung in die zumindest zweite Bewegungsrichtung entlang der Spindel bewegbar. Vorteilhafterweise weist der Aktor für die erste und die zumindest zweite Bewegungsrichtung je zumindest ein Wirkelement auf, um die Relativbewegung zwischen dem ersten und dem zweiten Einführelement der Einführvorrichtung zu bewirken. Hierdurch können die Bewegungen in die beiden Bewegungsrichtungen besonders präzise und effizient übertragen werden. Hierbei kann das Wirkelement von jedem, dem Fachmann für einsetzbar erachtetes Element gebildet sein, wie einem Zahnrad, einer Verzahnung, einem Gelenk und/oder einem Gewinde. Ferner ist es vorteilhaft, wenn das Wirkelement zumindest ein Gelenk umfasst, wodurch die jeweilige Bewegung in Bewegungsrichtung besonders gleichmäßig und stufenlos erfolgen kann.

In einer weiteren Ausgestaltung der Erfindung wird vorgeschlagen, dass eine Interaktion zwischen der Spindel und zumindest einem Wirkelement die Bewegung quer zur Longitudinalrichtung vermittelt, wodurch die Bewegung in die zumindest zweite Bewegungsrichtung konstruktiv einfach erfolgen kann. Zudem kann so die Umsetzung der Bewegung quer zu der Longitudinalrichtung in die Bewegung in Longitudinalrichtung einfach stattfinden. Die Interaktion zwischen der Spindel und dem Wirkelement kann vorteilhaft über ein Übertragungselement, wie einem Gewinde und insbesondere einem Innengewinde erfolgen. Dies erlaubt eine kompakte und einfache Konstruktion des Aktors bzw. der Freigabevorrichtung.

Ferner wird vorgeschlagen, dass zumindest ein Führungselement vorgesehen ist, das den Aktor während seiner Bewegung führt, wodurch die Bewegungen in die beiden Bewegungsrichtungen zuverlässig erfolgen können. Das Führungselement kann von jedem, dem Fachmann für sinnvoll erscheinendes Element gebildet sein, wie beispielsweise einer Öffnung, einem Kanal, einer Rinne, einer Wand, einer Stange und/oder einem Balken. Der Aktor und das Führungselement können mittels jeder Verbindungsart, wie einem Form-, Kraft-, und/oder Stoffschluss miteinander verbunden sein. Zudem kann eine relative Anordnung des Aktors und des Führungselements jede beliebige sein. Bevorzugt ist der Aktor an seinem proximalen Ende mit dem Führungselement verbunden. Für eine stabile Übertragung der Relativbewegung zwischen dem ersten und dem zweiten Einführelement ist das Führungselement zumindest mit einem Einführelement der Einführvorrichtung verbunden. Ist die Einführvorrichtung ein Katheter, so kann das betreffende Einführelement bevorzugt ein Außenschaft des Katheters sein.

In einer bevorzugten Ausführung ist das Führungselement an dem Körper, der von einem Gehäuse der Führungsvorrichtung gebildet ist, abgestützt, bzw. ist das Führungselement an dem Gehäuse, in dem der Aktor angeordnet ist, abgestützt. Dadurch können das Führungselement und damit der Aktor besonders stabil abgestützt werden. Hierzu kann das Gehäuse zumindest eine Führungsvorrichtung, wie eine Öffnung oder eine Kulissenbahn, aufweisen. Das Gehäuse kann insbesondere einen Handgriff der Einführvorrichtung bilden, wodurch sich eine kompakte Bauweise ergibt. Des Weiteren kann es vorteilhaft sein, wenn der Aktor, bevorzugt an seinem distalen Ende, am Gehäuse abgestützt und/oder befestigt ist. So ist der Aktor unverkippbar im Gehäuse befestigt, wodurch inhomogene Bewegungen vermieden werden können. Zudem kann es zu keinem Verklemmen der Bauteile kommen. Gemäß einer vorteilhaften Ausgestaltung kann das Führungselement eine Durchführung für eines der Einführelemente aufweisen. Dies erlaubt eine kompakte Anordnung, welche das durchgeführte Einführelement stabilisiert und schützt. Ist die Einführvorrichtung ein Katheter, so kann das betreffende Einführelement ein Innenschaft des, Katheters sein.

Eine einfache Bedienung kann vorteilhaft erreicht werden, wenn zumindest ein Wirkelement mit einem ersten Betätigungselement gekoppelt ist. Das erste Betätigungselement ist bevorzugt einstückig mit der Spindel ausgeführt. Hierbei soll unter "einstückig" verstanden werden, dass das Betätigungselement und die Spindel von demselben Bauteil gebildet sind und/oder nur unter Funktionsverlust zumindest eines der Bauteile voneinander getrennt werden können. Bauteile, Bauraum, Montageaufwand und Kosten können vorteilhaft eingespart werden, wenn die Spindel das erste Betätigungselement bildet. Zudem wird vorgeschlagen, dass das erste Betätigungselement aus einem Gehäuse, in dem der Aktor angeordnet ist, ragt, wodurch eine leichte und kontrollierte Bedienbarkeit der Freigabevorrichtung ermöglicht wird. Hierfür ist an dem aus dem Gehäuse ragenden Anteil der Spindel ein Griff angeordnet, mittels dem die Spindel in Umfangsrichtung gedreht werden kann. Eine bevorzugte Ausgestaltung besteht darin, dass das erste Bedienelement zu einer langsamen Freisetzung des Implantats dient, wodurch eine hohe Präzision bei der Positionierung des Implantats erreicht werden kann, statt wie im Stand der Technik ein Schieben und Zurückziehen der Einführelemente einzusetzen. In einer alternativen Ausgestaltung ist das erste Bedienelement bei der Bewegung relativ zu dem Gehäuse ortsfest angeordnet, wodurch die Freigabevorrichtung besonders kompakt gestaltet werden kann.

Eine bevorzugte Weiterbildung besteht darin, dass ein zweites Betätigungselement für die Relativbewegung zwischen dem ersten und dem zweiten Einführelement vorgesehen ist, das lösbar mit dem Aktor ausgebildet ist. Hierdurch kann das Implantat konstruktiv einfach unabhängig von dem Aktor freigegeben werden. Das zweite Bedienelement ist hierzu bevorzugt mit einem Einführelement der Einführvorrichtung verbunden und als ein Griff ausgebildet. Ist die Einführvorrichtung ein Katheter, so kann das betreffende Einführelement bevorzugt der Außenschaft des Katheters sein. Diese Verbindung wird konstruktiv einfach über das Führungselement vermittelt. Des Weiteren kann es vorteilhaft sein, wenn das zweite Bedienelement zu einer schnellen Freisetzung des Implantats dient, wodurch die Freigabevorrichtung besonders variabel eingesetzt werden kann. Die beiden Bedienelemente ermöglichen eine einfache Handhabung, insbesondere ein unkompliziertes Umschalten zwischen zwei Freigabemodi. Dies erlaubt eine unkomplizierte, einfache und schnelle Geschwindigkeitsregulierung bei der Freigabe des Implantats. Die Freigabe des Implantats wird präziser und schneller.

Es wird zudem vorgeschlagen, dass der Aktor zumindest eine Schiene aufweist, die in Richtung der zumindest zweiten Bewegungsrichtung angeordnet ist. Mittels dieser Schiene kann die Bewegung in Richtung der zumindest zweiten Bewegungsrichtung zusätzlich geführt werden, wodurch die Bewegung besonders gleichmäßig erfolgen kann. Des Weiteren ist es vorteilhaft, wenn entlang der Schiene zumindest ein Interaktionselement in Richtung der zumindest zweiten Bewegungsrichtung bewegbar ist, wodurch die Führung der Schiene konstruktiv einfach erfolgen kann. Das Interaktionselement kann von jedem, dem Fachmann für einsetzbar erachteten Element gebildet sein, wie einer Öffnung, einer Nut oder einem Gelenk. Bevorzugt ist das Interaktionselement an einem Schenkel des Aktors, beispielsweise ein einem Ende des Schenkels, angeordnet. Alternativ oder zusätzlich weist vorteilhaft zumindest ein Wirkelement das Interaktionselement auf und besonders bevorzugt ist das Interaktionselement einstückig mit zumindest einem Wirkelement ausgeführt.

Gemäß einer vorteilhaften Ausgestaltung kann der Aktor gelenkig miteinander verbundene Schenkel aufweisen, wodurch der Aktor mit einem geringen Gewicht realisiert werden kann. Ferner ist es in einer bevorzugten Ausgestaltung vorteilhaft, wenn die Schenkel in der Art eines Parallelogramms angeordnet sind. Hierdurch kann der Aktor konstruktiv einfach symmetrisch ausgebildet werden, wodurch auch die Bewegung in der zweiten Bewegungsrichtung mit konstantem Verstellgesetz auf die Bewegung in die erste Bewegungsrichtung übertragen werden kann. Der Aktor weist bevorzugt vier, insbesondere gleich lange, Schenkel auf, die eine Art Parallelogramm bilden, wobei die Spindel in einer Symmetrieachse des Parallelogramms liegt.

In einer alternativen Ausgestaltung wird vorgeschlagen, dass die Schenkel über zumindest ein Kreuzgelenk verbunden sind, wodurch eine Ausrichtung der Schenkel zueinander konstruktiv einfach und zuverlässig gestaltet werden kann. Hierdurch kann der Aktor Konstruktions- und Montageaufwand sparend mit zwei, insbesondere gleich langen, Schenkeln, die sich in einem Gelenke überschneiden, realisiert werden. Mittels dieser Ausgestaltung können die Schenkel mit einer sehr geringen Gefahr eines Verkantens gegeneinander bewegt werden.

Gemäß einem weiteren Aspekt der Erfindung wird eine Einführvorrichtung zum Einführen eines medizinischen Implantats vorgeschlagen, welches durch eine Relativbewegung zwischen einem ersten und einem zweiten Einführelement freisetzbar ist, umfassend eine Freigabevorrichtung zum Lösen des medizinisches Implantats, aufweisend einen Körper mit einem proximalen Ende, das im Benutzungszustand einem Anwender zugewandt ist und einem distalen Ende, das im Benutzungszustand vom Anwender entfernt ist, wobei zwischen dem proximalen und dem distalen Ende ein Aktor vorgesehen ist, wobei der Aktor zur Erzeugung einer gezielten Relativbewegung zwischen dem ersten und dem zweiten Einführelement der Einführvorrichtung eine erste und zumindest eine zweite Bewegungsrichtung aufweist, wobei bei der ersten Bewegungsrichtung das erste und das zweite Einführelemente in Longitudinalrichtung gegeneinander verschiebbar sind und wobei der Aktor bei der zumindest zweiten Bewegungsrichtung eine Bewegung quer zur Longitudinalrichtung bewirkt.

Durch die erfindungsgemäße Ausgestaltung kann eine Einführvorrichtung bereitgestellt werden, bei der große Kräfte unabhängig von der vom Anwender bzw. Arzt zu erbringenden Kraft einfach erzeugt werden können. Zudem weist sie eine einfache Handhabung und ein optimiertes Design auf. Die Freigabe des Implantats wird dadurch präziser und schneller. Ferner kann mittels des Aktors die Relativbewegung stabil und gleichmäßig realisiert werden. All dies resultiert in einer hohen Erfolgsrate der Implantation. Die Einführvorrichtung kann günstigerweise ein Katheter sein. Besonders vorteilhaft kann die Einführvorrichtung zur Montage und Freigabe einer Prothese, einer Herzklappe oder eines Stents eingesetzt werden.

Die Erfindung ist nachfolgend beispielhaft, anhand von in Zeichnungen dargestellten Ausfiihrungsbeispielen, näher erläutert. Es zeigen in schematischer Darstellung:
- Fig. 1: einen Schnitt durch ein günstiges Ausführungsbeispiel einer Einführvorrichtung und einer Freigabevorrichtung;
- Fig. 2: die Einführvorrichtung und Freigabevorrichtung aus Fig. 1 bei einer langsamen Freigabe eines Implantats durch Betätigung eines ersten Betätigungselements;
- Fig. 3: die Einführvorrichtung und Freigabevorrichtung aus Fig. 1 bei einer schnellen Freigabe des Implantats durch Betätigung eines zweiten Betätigungselements;
- Fig. 4: eine detaillierte Darstellung eines Aktors der Freigabevorrichtung der Fig. 1; und
- Fig. 5: eine alternative Freigabevorrichtung mit einem alternativen Aktor mit über ein Kreuzgelenk verbundenen Schenkeln.

In den Figuren sind funktionell gleiche oder gleich wirkende Elemente jeweils mit denselben Bezugszeichen beziffert. Die Figuren sind schematische Darstellungen der Erfindung. Sie bilden nicht spezifische Parameter der Erfindung ab. Weiterhin geben die Figuren lediglich typischen Ausgestaltungen der Erfindung wieder und sollen die Erfindung nicht auf die dargestellten Ausgestaltungen beschränken.

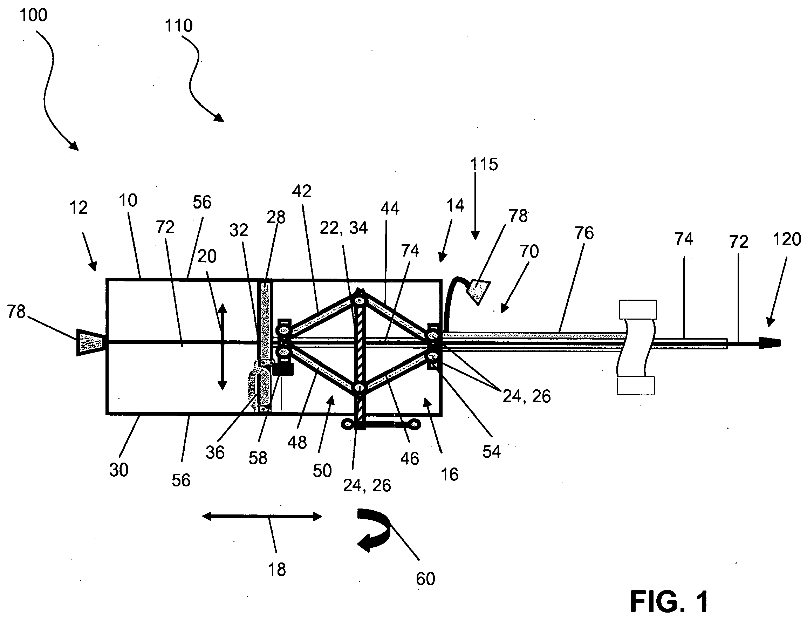

Figur 1 zeigt schematisch in einer Seitenansicht ein günstiges Ausführungsbeispiel einer Freigabevorrichtung 100 einer Einführvorrichtung 110 nach der Erfindung mit einem aufgeschnittenen Gehäuse 30, welches einen Handgriff der Einführvorrichtung 110 bildet.

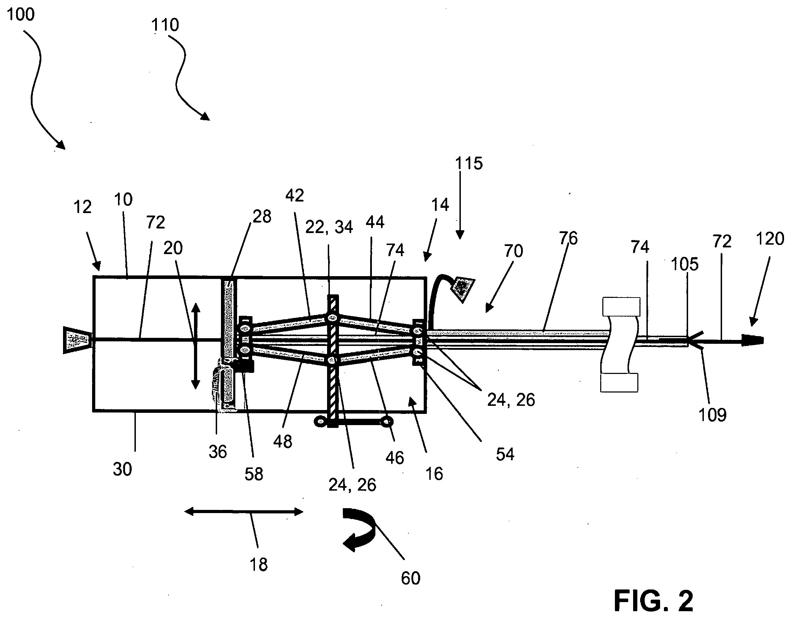

Die Einführvorrichtung 110 ist beispielsweise ein Katheter mit einem Schaftbereich 70 mit zwei koaxial angeordneten Einführelementen 72, 74, z.B. einem Innenschaft (Einführelement 72) und einem diesen umgebenden Außenschaft (Einführelement 74), welcher wiederum von einer Außenhülle 76 umgeben sein kann. Die Einführvorrichtung 110 ist in Anwenderbetrieb, also während einer Befestigung eines Implantats 105 an der Freigabevorrichtung 100 oder während der Implantation mit ihrem proximalen Ende 115 einem Anwender zugewandt. Das Implantat 105 ist an einem distalen Ende 120 des Schaftbereich 70 zwischen Innenschaft und Außenschaft platziert und soll am Implantationsort im tierischen oder menschlichen Körper freigegeben werden (siehe Fig. 2).

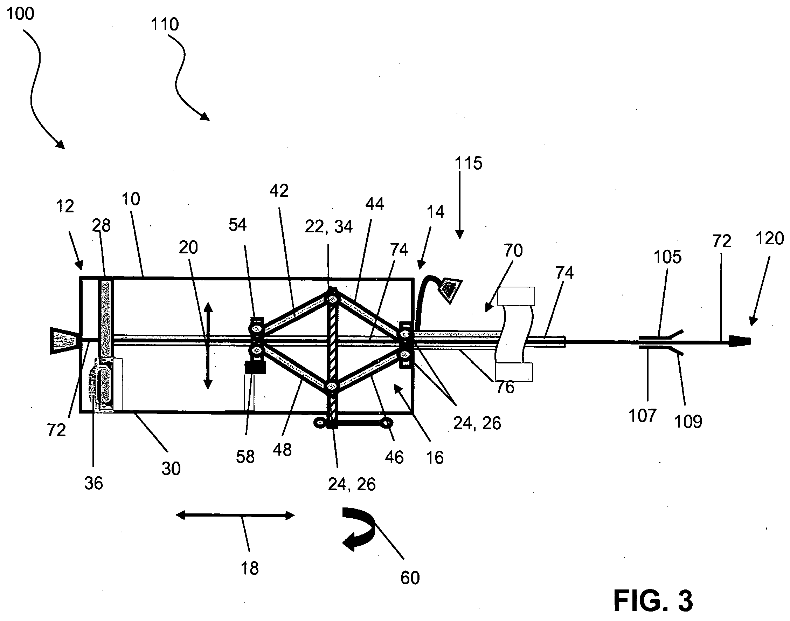

Die Freigabevorrichtung 100 dient zum Lösen des medizinischen Implantats 105 von der Einführvorrichtung 110. Das Implantat 105 ist an einem dem Gehäuse 30 entgegengesetzten Ende 120 des Schaftbereichs 70 beispielsweise in der Nähe einer Katheterspitze angeordnet. Das Implantat 105 ist beispielsweise um das innere Einführelement 72 gelegt und wird durch eine Relativbewegung zwischen dem ersten und dem zweiten Einführelement 72, 74 freigesetzt, wie dies teilweise in Fig. 2 und 3 angedeutet ist. Hierfür ist das Implantat 105 selbstexpandierend ausgeführt.

Die Freigabevorrichtung 100 umfasst einen Körper 10, ausgeführt als das Gehäuse 30, mit einem proximalen Ende 12, das im Benutzungszustand dem Anwender zugewandt ist und einem distalen Ende 14, das im Benutzungszustand vom Anwender entfernt ist. Zwischen dem proximalen und dem distalen Ende 12, 14 ist ein Aktor 16 vorgesehen. Der Aktor 16 weist zur Erzeugung einer gezielten Relativbewegung zwischen dem ersten und dem zweiten Einführelement 72, 74 der Einführvorrichtung 110 eine erste Bewegungsrichtung 18 und eine zweite Bewegungsrichtung 20 auf Bei der ersten Bewegungsrichtung 18 werden das erste und das zweite Einführelemente 72, 74 in Longitudinalrichtung 18 der Einführelemente 72, 74 gegeneinander verschoben. Bei der zweiten Bewegungsrichtung 20 hingegen bewirkt der Aktor 16 eine Bewegung quer bzw. senkrecht zur Longitudinalrichtung 18. Der Aktor 16 ist parallel bzw. nicht überschneidend zu den Einführelementen 72, 74 angeordnet.

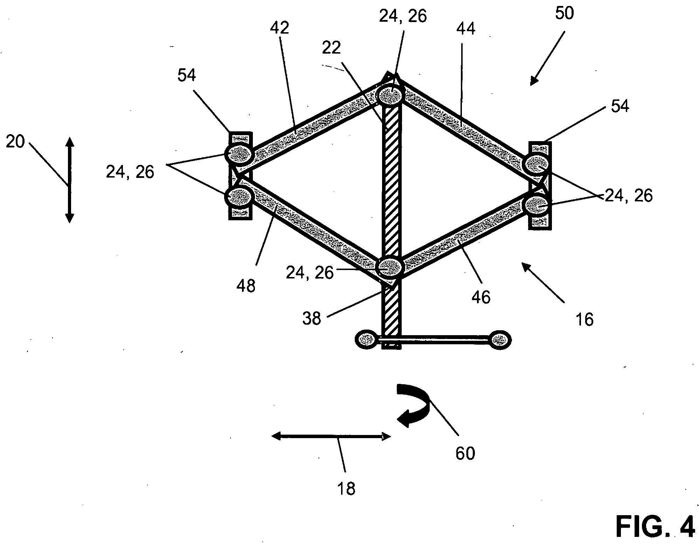

In der Fig. 4 ist der Aktor 16, der einen Scherenheber darstellt, im Detail gezeigt. Zur Vermittlung von Bewegungen in die beiden Bewegungsrichtungen 18, 20 weist der Aktor 16 vier gelenkig miteinander verbundene und gleich lange Schenkel 42, 44, 46, 48 auf, die in der Art eines Parallelogramms 50 angeordnet sind. Zudem weist der Aktor 16 hierfür mehrere bzw. sechs Wirkelemente 24 auf, die je ein Gelenk 26 umfassen. Hierbei sind jeweils zwei in Longitudinalrichtung 18 hintereinander angeordnete Schenkel 42 und 44 bzw. Schenkel 46 und 48 über ein Gelenk 26 verbunden. Zudem sind die Schenkelpaare 42, 44 und 46, 48 achsensymmetrisch zueinander angeordnet. Die zwei jeweils in der Richtung 20 senkrecht zur Longitudinalrichtung 18 übereinander angeordneten Schenkel 42 und 48 bzw. 44 und 46 sind jeweils über ein Verbindungselement 54 verbunden, das parallel zur Richtung 20 angeordnet ist. Zur Verbindung des jeweiligen Schenkels 42, 44, 46, 48 mit dem jeweiligen Verbindungselement 54 sind ebenso Gelenke 26 angeordnet. Somit weist jeder Schenkel 42, 44, 46, 48 an jedem seiner sich gegenüberliegenden Enden je ein Gelenk 26 auf. Das zum distalen Ende 14 des Körpers 10 weisende Verbindungselement 54 des Aktors 16 ist am distalen Ende 14 des Körpers 10 bzw. dem Gehäuse 30 befestigt. Das zum proximalen Ende 12 des Körpers 10 weisende Verbindungselement 54 des Aktors 16 ist lösbar an einem Führungselement 28 fixiert (vgl. Fig. 1).

Das Führungselement 28 ist als ein Balken ausgeführt, der das Gehäuse 30 in Richtung 20 durchspannt, und ist an dem Gehäuse 30 abgestützt bzw. in Longitudinalrichtung 18 verschieblich gelagert. Hierfür weist das Gehäuse 30 in seinen parallel zur Longitudinalrichtung 18 angeordneten Wandungen 56 nicht näher gezeigte Führungsvorrichtungen auf. An einer in Richtung des distalen Endes 14 des Körpers 10 orientierten Seite des Führungselements 28 ist das Einführelement 74 bzw. der Außenschaft befestigt. Zudem weist das Führungselement 28 eine Durchführung 32 für das Einführelemente 72 bzw. den Innenschaft auf. Dieser ist am proximalen Ende 12 des Körpers 10 bzw. dem Gehäuse 30 befestigt. Ein Lumen des Einführelements 72 (Innenschaft) kann mit einem am proximalen Ende 12 des Körpers angeordneten Entlüftungsventil 78 (auch bekannt als Luer-Lock) entlüftet und/oder gespült werden. Des Weiteren ist ein Entlüftungsventil 78 am proximalen Ende 115 der Einführvorrichtung 110 zum Entlüften eines Lumens der Außenhülle 78 angeordnet. Ferner weist die Einführvorrichtung 110 zwischen dem ersten und dem zweiten Einführelement 72, 74 nicht näher gezeigte Entlüftungslöcher auf.

Zudem weist der Aktor 16 eine Spindel 22 auf, die in Richtung der zweiten Bewegungsrichtung 20 koaxial zu den Gelenken 26 der Schenkelpaare 42, 44 bzw. 46, 48 angeordnet ist und eine Symmetrieachse des Aktors 16 bildet. Hierfür weisen die Gelenke 26 nicht näher gezeigte Übertragungselemente bzw. Gewindeabschnitte auf, in die ein Gewinde der Spindel 22 eingreift. Die Spindel 22 dient ferner als ein erstes Betätigungselement 34, wodurch die Wirkelemente 24 mit dem ersten Betätigungselement 34 gekoppelt sind. Ferner ragt das erste Betätigungselement 34 aus dem Gehäuse 30 und an seinem aus dem Gehäuse 30 ragenden Anteil ist ein Stabgriff zu dessen Drehung angeordnet. Um die Verbindung zwischen dem Führungselement 28 und dem Aktor 16 zu lösen, ist zwischen diesen Bauteilen ein Verriegelungselement 58 angeordnet. Dieses kann beispielsweise über ein mit dem Führungselement 28 verbundenes zweites Betätigungselement 36, ausgeführt als ein Bügelgriff, betätigt werden.

Das Gewinde der Spindel 22 und die Gewinde der Gewindeabschnitte der Gelenke 26 sind so aufeinander abgestimmt, dass sich bei einer Drehung der Spindel 22 in Umfangsrichtung 60 die Gelenke 26, die die Schenkelpaare 42, 44 bzw. 46, 48 verbinden, in Richtung 20 aufeinander zu bewegen, also entlang der Spindel 22 bewegbar sind. Dadurch bewirkt die Spindel 22 bzw. eine Interaktion zwischen der Spindel 22 und den Wirkelementen 24 die Bewegung quer bzw. senkrecht zu der Longitudinalrichtung 18. Wie in Fig. 2 gezeigt ist, wird hierdurch das Parallelogramm 50 in Richtung 20 gestaucht und in Longitudinalrichtung 18 gespreizt. Hierbei weist das Parallelogramm 50 in Richtung 20 beispielsweise einen Bewegungsraum von ca. 25 mm auf Bei dieser Gestaltänderung wird auch die Spindel 22 in Longitudinalrichtung 18 verschoben, hierfür weist das Gehäuse 30 in einem Bereich in dem die Spindel 22 bzw. das Bedienelement 34 aus dem Gehäuse 30 ragt eine sich in Longitudinalrichtung 18 erstreckende nicht näher gezeigte Aussparung auf.

Zeitgleich zum Annähern der Gelenke 26 in Richtung 20 erfolgt somit ein Entfernen der Verbindungselemente 54 in Longitudinalrichtung 18 voneinander. Durch die Verbindung des Aktors 16 mit dem Führungselement 28 durch das Verriegelungselement 58 wird auch das Führungselement 28 in Richtung des proximalen Endes 12 des Körpers 10 bewegt bzw. gedrückt. Wegen seiner Lagerung am Gehäuse 30 führt das Führungselement 28 den Aktor 16 während seiner Bewegung. Die Bewegung des Führungselements 28 führt zu einer Bewegung des Einführelements 74 in Richtung des proximalen Endes 12 und folglich zu einer Freilegung des Implantats 105 am distalen Ende 120 der Einführvorrichtung 110. Dies erfolg erst an einem distalen Ende 109 des Implantats 105. Das Bedienelement 34 dient zu einer langsamen Freisetzung des Implantats 105. Grundsätzlich kann das Implantat 105 ausschließlich über die langsame Freisetzung platziert werden. Hierfür müssen Maße des Aktors 16 an eine Freigabegeometrie der Einführvorrichtung und/oder eine Länge des Implantats 105 angepasst sein. Dies wird der Fachmann anhand seiner Fachkenntnis selbsttätig tun.

Zu einer schnellen Freisetzung des Implantats 105 dient das zweite Betätigungselement 36. Hierbei kann die schnelle Freisetzung nach einer langsamen und teilweisen Freisetzung des Implantat 105 zu dessen vollständiger Freisetzung erfolgen oder das Implantat 105 kann ausschließlich mit der schnellen Freisetzung platziert werden. Mittels des zweiten Betätigungselements 36 werden der Aktor 16 und das Führungselementen 28 durch Lösen des Verriegelungselements 58 voneinander entkoppelt. Hiernach kann die Führungsvorrichtung 28 und damit das Einführelement 74 (Außenschaft), beispielsweise durch manuelles Ziehen des Betätigungselements 36, in Longitudinalrichtung 18 zum proximalen Ende 12 des Körpers 10 verschoben werden. Hierdurch wird auch das proximale Ende 107 des Implantats 105 freigelegt. Diese Situation ist in Fig. 3gezeigt. Anschließend wird die Einführvorrichtung 110 zurückgezogen und das Implantat 105 verbleibt am Implantationsort.

Die Mechanismen der langsamen und schnellen Freigabe und die dazu verwendeten Bauelemente können auch zu einer Befestigung des Implantats 105 vor einer Einführung der Einführvorrichtung 110 in den Körper verwendet werden. Hierbei kann der Innenschaft am distalen Ende 120 wie oben beschrieben freigelegt werden. Nach der Positionierung des Implantats 105 am Innenschaft kann der Außenschaft durch schieben des Führungselements 28 in Richtung 18 des distalen Endes 14 des Körpers 10 zum Abdecken des Implantats 105 bewegt werden. Eine vollständige Abdeckung und Bewegung des Außenschafts in Richtung des distalen Endes 120 kann nach der Verriegelung des Führungselements 28 und des Aktors 16 mittels des Verriegelungselements 58 mittels des Aktors 16 erfolgen. Hierbei wird die Spindel 22 entgegen der Umfangsrichtung 60 bewegt, wodurch das Parallelogramm 50 in Richtung 20 gespreizt und in Longitudinalrichtung 18 gestaucht wird, wodurch das Führungselement 28 und der Außenschaft in Richtung 18 der distalen Enden 14, 120 bewegt werden (nicht gezeigt).

Eine Länge jedes Körpers 10 bzw. der Bewegungsspielraum des Aktors 16 sind zweckmäßigerweise so bemessen, dass dieser mindestens so lang ist wie die freizugebende Länge des Implantats 105. Im Falle eines Katheters als Einführvorrichtung 110 mit einem Stent als Implantat 105 kann in der Praxis der Stent anfangs mit langsamer Geschwindigkeit bis zu einer gewissen Länge freigesetzt und somit sehr präzise positioniert werden. Danach kann der Stent vollständig mit höherer Geschwindigkeit freigesetzt werden. Das langsame Freigeben eignet sich besonders für den Beginn der Implantatsfreigabe am Implantationsort.

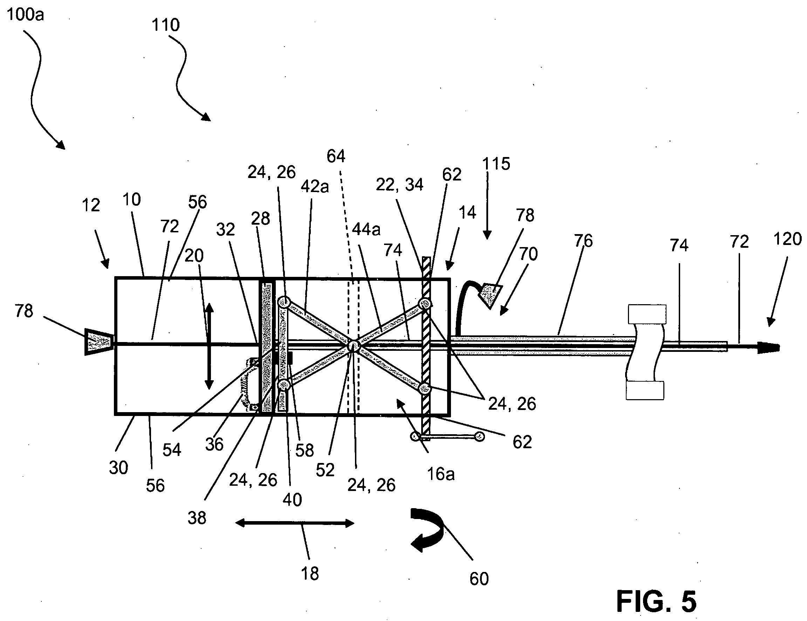

In Fig. 5 ist ein alternatives Ausführungsbeispiel der Freigabevorrichtung 100 dargestellt. Im Wesentlichen sind gleich bleibende Bauteile, Merkmale und Funktionen grundsätzlich mit den gleichen Bezugszeichen beziffert. Zur Unterscheidung des Ausführungsbeispiels der Fig. 5 zu dem in den Fig. 1 bis 4 sind jedoch den Bezugszeichen der in dem Ausführungsbeispiel der Fig. 5 abweichenden ausgeführten Bauteile der Buchstabe a hinzugefügt. Die nachfolgende Beschreibung beschränkt sich im Wesentlichen auf die Unterschiede zu dem Ausführungsbeispiel in den Fig. 1 bis 4, wobei bezüglich gleich bleibender Bauteile, Merkmale und Funktionen auf die Beschreibung des Ausführungsbeispiels in den Fig. 1 bis 4 verwiesen werden kann.

Fig. 5 zeigt eine alternative Freigabevorrichtung 100a mit einem weiteren alternativen Aktor 16a, der in einem Körper 10, der von einem Gehäuse 30 gebildet ist und ein proximales Ende 12 und ein distales Ende 14 aufweist, angeordnet ist. Der Aktor 16a weist zur Erzeugung einer gezielten Relativbewegung zwischen einem ersten und einem zweiten Einführelement 72, 74 einer Einführvorrichtung 110 eine erste und eine zweite Bewegungsrichtung 18; 20 auf, wobei bei der ersten Bewegungsrichtung 18 das erste und das zweite Einführelemente 72, 74 in Longitudinalrichtung 18 gegeneinander verschiebbar sind und wobei der Aktor 16a bei der zumindest zweiten Bewegungsrichtung 20 eine Bewegung quer zur Longitudinalrichtung 18 bewirkt. Hierzu weist der Aktor 16a zwei gleich lange Schenkel 42a, 44a auf, die über ein Kreuzgelenk 52 verbunden sind. Die Schenkel 42a, 44a sind an Enden, die in Richtung 20 zum distalen Ende 14 des Körpers 10 angeordnet sind, mit je einem Wirkelement 24, das ein Gelenk 26 umfasst, mit einer Spindel 22 verbunden. Zu einer Lagerung des Aktors 16a durchgreift die Spindel 22, die ein erstes Bedienelement 34 darstellt, an ihren zwei Enden nicht im Detail gezeigte Gewindebohrungen 62, wobei diese in Wandungen 56 des Gehäuses 30, die in Longitudinalrichtung 18 orientiert sind, eingebracht sind. Alternativ kann der Aktor 16a über ein Lagerelement 64 am Gehäuse 30 abgestützt sein, das in nicht näher gezeigten Führungsvorrichtungen des Gehäuses 30 geführt ist.

An Enden der Schenkel 42a, 44a, die in Richtung 20 zum proximalen Ende 12 des Körpers 10 angeordnet sind, sind diese über ein parallel zur Spindel 22 angeordnetes Verbindungselement 54 verbunden. Hierzu ist je Schenkel 42a, 44a ein Wirkelement 24, das ein Gelenk 26 umfasst, vorgesehen. Das Verbindungselement 54 bzw. der Aktor 16a umfassen eine Schiene 38 die in Richtung der zweiten Bewegungsrichtung 20 und damit parallel zur Spindel 22 angeordnet ist. Entlang der Schiene 38 ist ein Interaktionselement 40 in Richtung der zweiten Bewegungsrichtung 20 bewegbar. Das Interaktionselement 40 ist einstückig mit einem Wirkelement 24 bzw. einem Gelenk 26 ausgeführt, wodurch die Schiene 38 eine Bewegung von mindestens einem Wirkelement 24 gewährleistet. Zudem ist das Verbindungselement 54 über ein Verriegelungselement 58 lösbar an einem Führungselement 28 fixiert. Wird die Spindel 22 nun in Umfangsrichtung 60 gedreht, bewegen sich die Gelenke 26 an den distalen Enden der Schenkel 42a, 44a aufeinander zu, wodurch die Schenkel 42a, 44a um das Kreuzgelenk 52 geschwenkt werden. Hierdurch bewegen sich auch die proximalen Enden der Schenkel 42a, 44a aufeinander zu und das Verbindungselement 54 mit dem Führungselement 28 werden in Richtung 18 zum proximalen Ende 12 des Körpers 10 gedrückt. Dadurch wird auch das am Führungselement 28 befestigte Einführelement 74 relativ zum Einführelement 72 bewegt, wodurch ein hier nicht gezeigtes Implantat an einem distalen Ende 120 der Einführvorrichtung 110 freigelegt oder ein Ende langsam freigesetzt wird. Bei dieser Konstruktion des Aktors 16a ist die Spindel 22 gegenüber dem Gehäuse 30 ortsfest angeordnet, sie bewegt sich bei der Schwenkbewegung der Schenkel 42a, 44a nicht in Longitudinalrichtung 18.

Eine schnelle Freisetzung erfolgt wie im Ausführungsbeispiel der Fig. 1 bis 4 beschrieben über eine Entkopplung des Aktors 16a und des Führungselements 28 mittels eines zweiten Bedienelements 36.

## Patentansprüche

1. Freigabevorrichtung (100, 100a) zum Lösen eines medizinischen Implantats (105) von einer Einführvorrichtung (110), bei welcher das Implantat (105) durch eine Relativbewegung zwischen einem ersten und einem zweiten Einführelement (72; 74) freisetzbar ist, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12; 14) ein Aktor (16, 16a) vorgesehen ist, wobei der Aktor (16, 16a) zur Erzeugung einer gezielten Relativbewegung zwischen dem ersten und dem zweiten Einführelement (72; 74) der Einführvorrichtung (110) eine erste und zumindest eine zweite Bewegungsrichtung (18; 20) aufweist, wobei bei der ersten Bewegungsrichtung (18) das erste und das zweite Einführelemente (72; 74) in Longitudinalrichtung (18) gegeneinander verschiebbar sind und wobei der Aktor (16, 16a) bei der zumindest zweiten Bewegungsrichtung (20) eine Bewegung quer zur Longitudinalrichtung (18) bewirkt, **dadurch gekennzeichnet, dass**, der Aktor (16, 16a) zumindest eine Spindel (22) aufweist, die in Richtung der zumindest zweiten Bewegungsrichtung (20) angeordnet ist und/oder die die Bewegung quer zu der Longitudinalrichtung (18) bewirkt.

2. Freigabevorrichtung nach Anspruch 1, wobei der Aktor (16, 16a) für die erste und die zumindest zweite Bewegungsrichtung (18; 20) je zumindest ein Wirkelement (24) aufweist, um die Relativbewegung zwischen dem ersten und dem zweiten Einführelement (72; 74) der Einführvorrichtung (110) zu bewirken.

3. Freigabevorrichtung nach Anspruch 2, wobei das Wirkelement (24) zumindest ein Gelenk (26) umfasst.

4. Freigabevorrichtung nach einem der Ansprüche 1 bis 3, wobei eine Interaktion zwischen der Spindel (22) und zumindest einem Wirkelement (24) die Bewegung quer zur Longitudinalrichtung (18) vermittelt.

5. Freigabevorrichtung nach einem der vorhergehenden Ansprüche, wobei zumindest ein Führungselement (28) vorgesehen ist, das den Aktor (16, 16a) während seiner Bewegung führt.

6. Freigabevorrichtung nach Anspruch 5, wobei das Führungselement (28) an zumindest einem Gehäuse (30), in dem der Aktor (16, 16a) angeordnet ist, abgestützt ist.

7. Freigabevorrichtung nach zumindest einem der Ansprüche 5 oder 6, wobei das Führungselement (28) eine Durchführung (32) für eines der Einführelemente (72) aufweist.

8. Freigabevorrichtung nach einem der vorhergehenden Ansprüche, wobei zumindest ein Wirkelement (24) mit einem ersten Betätigungselement (34) gekoppelt ist, das aus einem Gehäuse (30), in dem der Aktor (16, 16a) angeordnet ist, ragt und/oder zu einer langsamen Freisetzung des Implantats (105) dient.

9. Freigabevorrichtung nach einem der vorhergehenden Ansprüche, wobei ein zweites Betätigungselement (36) für die Relativbewegung zwischen dem ersten und dem zweiten Einführelement (72; 74) vorgesehen ist, das lösbar mit dem Aktor (16, 16a) ausgebildet ist und/oder das zu einer schnellen Freisetzung des Implantats (105) dient.

10. Freigabevorrichtung zumindest nach Anspruch 2, wobei der Aktor (16a) zumindest eine Schiene (38) aufweist, die in Richtung der zumindest zweiten Bewegungsrichtung (20) angeordnet ist und/oder entlang der zumindest ein Interaktionselement (40) in Richtung der zumindest zweiten Bewegungsrichtung (20) bewegbar ist.

11. Freigabevorrichtung nach einem der vorhergehenden Ansprüche, wobei der Aktor (16, 16a) gelenkig miteinander verbundene Schenkel (42, 42a; 44, 44a; 46; 48) aufweist.

12. Freigabevorrichtung nach Anspruch 11, wobei die Schenkel (42; 44; 46; 48) in der Art eines Parallelogramms (50) angeordnet sind.

13. Freigabevorrichtung nach Anspruch 11, wobei die Schenkel (42a; 44a) über zumindest ein Kreuzgelenk (52) verbunden sind.

14. Einführvorrichtung (110) zum Einführen eines medizinischen Implantats (105), welches durch eine Relativbewegung zwischen einem ersten und einem zweiten Einführelement (72; 74) freisetzbar ist, umfassend eine Freigabevorrichtung (100, 100a) zum Lösen des medizinisches Implantats (105 nach einem der vorhergehenden Ansprüche, umfassend einen Körper (10) mit einem proximalen Ende (12), das im Benutzungszustand einem Anwender zugewandt ist und einem distalen Ende (14), das im Benutzungszustand vom Anwender entfernt ist, wobei zwischen dem proximalen und dem distalen Ende (12; 14) ein Aktor (16, 16a) vorgesehen ist, wobei der Aktor (16, 16a) zur Erzeugung einer gezielten Relativbewegung zwischen dem ersten und dem zweiten Einführelement (72; 74) der Einführvorrichtung (110) eine erste und zumindest eine zweite Bewegungsrichtung (18, 20) aufweist, wobei bei der ersten Bewegungsrichtung (18) das erste und das zweite Einführelemente (72; 74) in Longitudinalrichtung (18) gegeneinander verschiebbar sind und wobei der Aktor (16, 16a) bei der zumindest zweiten Bewegungsrichtung (20) eine Bewegung quer zur Longitudinalrichtung (18) bewirkt.

## Claims

1. A release device (100) for disengaging a medical implant from an insertion device (110), in which the implant is releasable by a relative movement between a first insertion element and a second insertion element (52, 54), comprising a body (10) having a proximal end (12), which faces towards a user in the usage state, and a distal end (14), which is remote from the user in the usage state, wherein a toothed rack (11) having a first speed range (16) and a second speed range (18) is provided between the proximal end and the distal end (12, 14), wherein the toothed rack (11) is provided to generate a targeted relative movement between the first insertion element and the second insertion element (52, 54) of the insertion device (110), **characterised in that**
the first speed range and the second speed range (16, 18) are situated on opposing regions of the toothed rack (11).

2. The release device according to Claim 1, wherein the first and the second speed range (16, 18) are each situated along a longitudinal extension (L) of the toothed rack (11).

3. The release device according to one of the preceding claims, wherein an action element (26, 28) is assigned in each case to the respective speed range (16, 18) and cooperates with the respective speed range (16, 18) in order to cause the relative movement between the first insertion element and the second insertion element (52, 54) of the insertion device (10).

4. The release device according to one of the preceding claims, wherein the first speed range and the second speed range (16, 18) are rows of teeth (16a, 18b), wherein the speed ranges (16, 18) have different tooth intervals from one another.

5. The release device according to one of the preceding claims, wherein the action element (26, 28) is fixed in relation to a housing (30), which is situated at least around the toothed rack (11).

6. The release device according to Claim 5, wherein the action element (26, 28) is coupled to an actuation element (26a, 28b), which protrudes out of the housing (30).

7. The release device according to one of the preceding claims, wherein the toothed rack (11) has a feedthrough (20) for one of the insertion elements (52).

8. The release device according to one of the preceding claims, wherein the action element (26, 28) has a gearwheel.

9. The release device according to one of Claims 5 to 8, wherein the inner insertion element (52) has a deaeration valve (32).

10. An insertion device (110) for inserting a medical implant, which is releasable by a relative movement between a first insertion element and a second insertion element (26, 28), comprising a release device (100) for disengaging the medical implant according to one of the preceding claims, comprising a body (10) having a proximal end (12), which faces towards a user in the usage state, and a distal end (14), which is remote from the user in the usage state, wherein a toothed rack (11) having a first speed range (16) and a second speed range (18) is provided between the proximal end and the distal end (12, 14), wherein the toothed rack (11) is provided to generate a targeted relative movement between the first insertion element and the second insertion element (52, 54) of the insertion device (110).

11. The insertion device according to Claim 10, wherein an airlock device (40), which is connected to an outer of the insertion elements (54), is situated at the distal end (12).

12. The insertion device according to Claim 10 or 11, wherein an inner insertion element (52) is guided by the toothed rack (11).

13. The insertion device according to one of Claims 11 to 12, wherein the toothed rack (11) is fixed on the airlock device (40).

14. The insertion device according to one of Claims 10 to 13, wherein an action element (26, 28) is assigned in each case to the respective speed range (16, 18) and cooperates with the respective speed range (16, 18) in order to cause the relative movement between the first insertion element and the second insertion element (52, 54) of the insertion device (110).

## Revendications

1. Dispositif de libération (100) pour enlever un implant médical d'un dispositif d'introduction (110), dans lequel l'implant peut être libéré par un mouvement relatif entre un premier et un deuxième éléments d'introduction (52, 54), comprenant un corps (10) avec une extrémité proximale (12), qui est orientée face à un utilisateur dans l'état d'utilisation, et avec une extrémité distale (14), qui est éloignée de l'utilisateur dans l'état d'utilisation, dans lequel une crémaillère (11) avec un premier domaine de vitesse (16) et un deuxième domaine de vitesse (18) est prévue entre les extrémités proximale et distale (12, 14), dans lequel la crémaillère (11) est prévue pour la création d'un mouvement relatif ciblé entre les premier et deuxième éléments d'introduction (52, 54) du dispositif d'introduction (110), **caractérisé en ce que** les premier et deuxième domaines de vitesse (16, 18) sont agencés sur des domaines de la crémaillère (11) situés l'un en face de l'autre.

2. Dispositif de libération selon la revendication 1, dans lequel les premier et deuxième domaines de vitesse (16, 18) sont agencés chaque fois le long d'une extension longitudinale (L) de la crémaillère (11).

3. Dispositif de libération selon l'une des revendications précédentes, dans lequel un élément actif (26, 28) est associé à chaque domaine de vitesse (16, 18), qui agit conjointement avec le domaine de vitesse (16, 18) correspondant afin de provoquer le mouvement relatif entre les premier et deuxième éléments d'introduction (52, 54) du dispositif d'introduction (110).

4. Dispositif de libération selon l'une des revendications précédentes, dans lequel les premier et deuxième domaines de vitesse (16, 18) sont constitués d'une rangée de dents (16a, 18b), où les domaines de vitesse (16, 18) présentent des espaces entre les dents différents l'un de l'autre.

5. Dispositif de libération selon l'une des revendications précédentes, dans lequel l'élément actif (26, 28) est fixé en face d'un boîtier (30) qui est disposé au moins autour de la crémaillère (11).

6. Dispositif de libération selon la revendication 5, dans lequel l'élément actif (26, 28) est couplé avec un élément d'actionnement (26a, 28b) qui dépasse du boitier (30).

7. Dispositif de libération selon l'une des revendications précédentes, dans lequel la crémaillère (11) présente un passage (20) pour l'un des éléments d'introduction (52).

8. Dispositif de libération selon l'une des revendications précédentes, dans lequel l'élément actif (26, 28) comprend un engrenage.

9. Dispositif de libération selon l'une des revendications 5 à 8, dans lequel l'élément d'introduction interne (52) présente une soupape de mise à l'air (32).

10. Dispositif d'introduction (110) pour l'introduction d'un implant médical, lequel peut être libéré par un mouvement relatif entre un premier et un deuxième éléments d'introduction (26, 28), comprenant un dispositif de libération (100) pour l'enlèvement de l'implant médical, selon l'une des revendications précédentes, comprenant un corps (10) avec une extrémité proximale (12) qui est orientée vers un utilisateur dans l'état d'utilisation et une extrémité distale (14) qui est éloignée de l'utilisateur dans l'état d'utilisation, dans lequel une crémaillère (11) avec un premier domaine de vitesse (16) et un deuxième domaine de vitesse (18) est prévue entre les extrémités proximale et distale (12, 14), dans lequel, pour la création d'un mouvement relatif ciblé, la crémaillère (11) est prévue entre les premier et deuxième éléments d'introduction (52, 54) du dispositif d'introduction (110).

11. Dispositif d'introduction selon la revendication 10, dans lequel, un dispositif de verrouillage (40) est agencé à l'extrémité distale (12), qui est relié avec l'un des éléments d'introduction externe (54).

12. Dispositif d'introduction selon les revendications 10 ou 11, dans lequel un élément d'introduction interne (52) est guidé par la crémaillère (11).

13. Dispositif d'introduction selon l'une des revendications 11 à 12, dans lequel la crémaillère (11) est fixée sur le dispositif de verrouillage (40).

14. Dispositif d'introduction selon l'une des revendications 10 à 13, dans lequel, chaque fois, un élément actif (26, 28) est associé à un domaine de vitesse respectif (16, 18), qui agit conjointement avec le domaine de vitesse (16, 18) correspondant, afin de provoquer le mouvement relatif entre les premier et deuxième éléments d'introduction (52, 54) du dispositif d'introduction (110).
